(19) Europäisches Patentamt
European Patent Office
Office européen des brevets

(11) Numéro de publication: 0 202 986 B1

(12) **FASCICULE DE BREVET EUROPEEN**

(45) Date de publication de fascicule du brevet: **15.07.92**

(51) Int. Cl.$^5$: **A61K 31/34**

(21) Numéro de dépôt: **86400867.7**

(22) Date de dépôt: **22.04.86**

(54) **Utilisation du "naftidrofuryl" pour le traitement des neuropathies.**

(30) Priorité: **23.04.85 FR 8506132**

(43) Date de publication de la demande:
**26.11.86 Bulletin 86/48**

(45) Mention de la délivrance du brevet:
**15.07.92 Bulletin 92/29**

(84) Etats contractants désignés:
**AT BE CH DE FR GB IT LI LU NL SE**

(56) Documents cités:
**FR-M- 3 843**

**The Merck Manual of Diagnosis and Therapy, 15me Edition, 1987, pages 1069-1074 et 1443-1445.**

**La Prensa Medica Argentina, vol. 58, no. 21, 23.07.71., pages 1067-1070.**

**British Medical Journal, vol. 2, no. 6153, 16.12.78., pages 1678-1679.**

**J. Gerontol., vol. 33, no. 2, mars 1978, pages 217-223.**

**Brain Research, vol. 279, no. 1-2, 21.11.83., pages 217-228, Elsevier Science Publishers B.V.**

**Thérapeutique, vol. 49, no. 9, novembre 1973, pages 567-576.**

(73) Titulaire: **LIPHA, LYONNAISE INDUSTRIELLE PHARMACEUTIOUE**
**34, rue Saint Romain Boîte Postale 8481**
**F-69359 Lyon Cedex 08(FR)**

(72) Inventeur: **Mosnier, Michel**
**3 bis Villa des Fleurs**
**F-94220 Charenton(FR)**
Inventeur: **Grand, Marcel**
**15, rue du Professeur Nicolas**
**F-69008 Lyon(FR)**

(74) Mandataire: **Le Guen, Gérard et al**
**CABINET LAVOIX 2, place d'Estienne d'Orves**
**F-75441 Paris Cédex 09(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services

## Description

La présente invention concerne l'utilisation du tétrahydro-α-(naphtyl-1 méthyl) furanne-2 propanoate de N,N - diéthylamino - 2 éthyle et de ses sels d'addition dans la préparation d'un médicament pour une application thérapeutique nouvelle relevant du traitement des neuropathies d'origines variées.

Il est connu que la fibre nerveuse est douée d'une grande faculté de régénération à condition que les corps cellulaires des neurones n'aient pas dégénéré ou été détruits. Ce phénomène physiologique de régénération axonale vise à permettre une reprise de l'activité fonctionnelle, néanmoins cette régénération est relativement lente, et compte tenu de la longueur du segment à régénérer, la récupération fonctionnelle peut prendre une à plusieurs années selon la nature du nerf lésé.

Dans l'ensemble des affections du système nerveux, les neuropathies à dégénérescence axonale représentent par leur fréquence une cause majeure de souffrance et d'invalidité dans le monde, ce qui explique que l'on ait souvent tenté d'accélérer la repousse des nerfs. Divers agents ont été utilisés dans ce but avec des succès variés mais se sont le plus souvent révélés toxiques aux doses permettant d'obtenir une action positive sur la croissance nerveuse.

Par les brevets d'invention n° 1.289.597, en particulier l'addition 83.555 et le brevet français 1.363.948, on connait les constantes physiques et des modes d'obtention du tétrahydro -α-(naphtyl-1 méthyl) furanne-2 propanoate de N,N - diéthylamino-2 éthyle, représenté par la formule

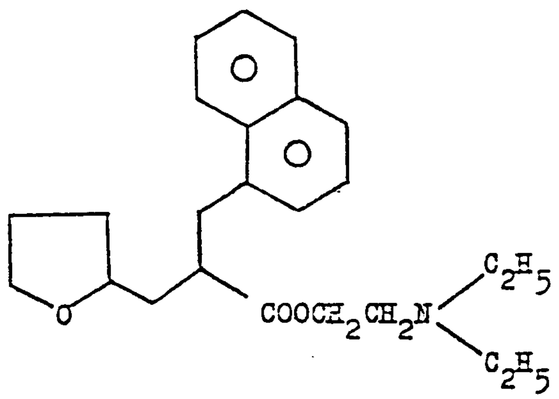

En outre, par le brevet spécial de médicament n° 3.843 M, on sait que, le tétrahydro-α-(naphtyl-1 méthyl) furanne-2 propanoate de N,N - diéthylamino-2 éthyle, communément désigné "Naftidrofuryl", ainsi que ses sels d'acides minéraux ou organiques sont doués de propriétés antispasmodiques et vasodilatatrices. Parmi ces composés l'oxalate de "Naftidrofuryl" constitue le principe actif de médicaments utilisés en médecine humaine pour leur action vasodilatatrice.

Le Naftidrofuryl a fait l'objet de très nombreux travaux scientifiques dans divers pays, depuis une vingtaine d'années, il est également connu comme un activateur du métabolisme du glucose dans la cellule cérébrale et comme un vasodilatateur cérébral. Des études récentes (S. Kobayashi et al., Arzneimittel Forschung 34, 1984, p. 1580-1583) ont montré son effet favorable sur la circulation cérébrale après infarctus cérébral aigu. Le Naftidrofuryl améliore le débit cérébral régional dans les zones cérébrales ischémiées. Le traitement des ulcères veineux avec le Naftidrofuryl a été étudié au Centre Hospitalier Régional de Reims J. Mal. Vasc. (France), 1984, 9 (2), p. 133-6, et l'efficacité du Naftidrofuryl dans l'occlusion des artères contrôlée par U. Maass et al, Medizinische Hochschule Hannover, Dtsch. Med. Wochenschr. (R.F.A.) 11 mai 1984, 109, p.745-50, ainsi que par Trubestein G et al, Angiology (U.S.) nov. 1984, 35, p. 701-8.

Il a également été démontré que le Naftidrofuryl présentait un intérêt en psychopharmacologie gériatrique (R.J. Branconnier et al, J. Gérontol., vol. 33, N° 2, mars 1978, pages 217-223) et qu'il était efficace en neurologie dans diverses pathologies d'origine artérielle (B.H. AHUMADA, Prensa Med. Argent ; vol. 58, N° 21, 23 juillet 1971, pages 1067-1070).

On vient de découvrir de façon tout à fait surprenante et inattendue que le Naftidrofuryl et ses sels d'addition exercent un effet complètement distinct des actions déjà connues, sur la croissance des cellules nerveuses et pouvait ainsi constituer un facteur de croissance nerveuse hautement significatif.

La présente invention concerne donc l'utilisation du tétrahydro-α-(naphtyl-1 méthyl) furanne-2 propanoate de N,N-diéthylamino-2 éthyle, "Naftidrofuryl", pour l'obtention d'un médicament destiné à favoriser la régénération des fibres nerveuses, notamment en vue du traitement des neuropathies.

Cet effet est sans aucune relation avec les effets du Naftidrofuryl déjà établis. Cette nouvelle propriété confère à ces composés une aptitude à régénérer la fibre nerveuse, ce qui rend particulièrement intéressante leur utilisation comme principes actifs de médicaments trouvant leur application dans cet autre domaine thérapeutique qui est celui du traitement des neuropathies d'origines variées, comme par exemple les neuropathies post-traumatique, les neuropathies diabétiques, certaines neuropathies toxiques comme les polynévrites éthyliques ou encore les neuropathies d'origine bactérienne ou virale et les neuropathies dégénératives.

Le Naftidrofuryl et ses sels d'addition sont également utiles pour rétablir ou améliorer la perception douloureuse au niveau cutané après intervention chirurgicale.

On englobe dans la dénomination "Naftidrofuryl", aussi bien le composé racémique que les diastéréo isomères A et B ou les antipodes optiques d et l des diastéréo isomères A et B. La dénomination "Naftidrofuryl" englobe également les diastéréo isomères A et B en mélange en proportions quelconques.

Parmi les sels d'addition du Naftidrofuryl avec les acides pharmaceutiquement acceptables, on peut citer avec intérêt, les sels formés avec les acides minéraux : chlorhydrique, bromhydrique, iodhydrique, nitrique, sulfurique, phosphorique, et avec les acides organiques : formique, benzoïque, fumarique, succinique, tartrique, citrique, oxalique, glyoxylique, aspartique, alcanesulfoniques et arylsulfoniques. Les sels de polyacides du Naftidrofuryl tels que l'oxalate, le fumarate et le citrate peuvent présenter un intérêt particulier en tant que principe actif dans la fabrication de médicaments régénérant les fibres nerveuses.

Les médicaments fabriqués en vue de la nouvelle application thérapeutique contiennent une quantité neurologiquement efficace de Naftidrofuryl ou d'un de ses sels d'addition en mélange ou en association avec un véhicule ou un excipient inerte, non toxique pharmaceutiquement acceptable.

Les médicaments pourront être fabriqués sous diverses formes pharmaceutiques, on citera celles qui conviennent à l'administration buccale comme les comprimés nus ou enrobés, les dragées, les microgranules, les comprimés à plusieurs noyaux, les gélules, les capsules, les poudres ou les granulés.

Celles qui conviennent à l'administration par voie parentérale comprennent les solutés ou suspensions injectables réparties en ampoules, en flacons multi-doses ou en seringues prêtes à l'injection

On citera aussi les formes appropriées pour l'administration par voie rectale comme les suppositoires ou les capsules rectales.

La toxicité du Naftidrofuryl et de ses sels d'addition est faible, ainsi la dose léthale DL 50 du Naftidrofuryl oxalate sur la souris per os est de 365 mg/kg et intra-péritonéale de 225 mg/kg et sur le rat P.O de 1.732 mg/kg et en I.P. de 136 mg/kg.

La dose journalière en principe actif par voie orale est comprise entre 300 mg et 1,5g, et par voie veineuse éventuellement en perfusion, elle est comprise entre 400 et 1.200 mg, de préférence 800 à 1200 mg.

Les médicaments selon l'invention renferment de 0,100 à 1g de Naftidrofuryl ou d'un de ses sels d'addition et de préférence 200 à 400 mg par dose unitaire.

Ces médicaments contenant comme principe actif le Naftidrofuryl ou un de ses sels d'addition peuvent en outre inclure un autre principe actif à action synergique, notamment ceux qui améliorent la résorption ou la diffusion du principe actif dans l'organisme.

Les exemples suivants démontrent la nouvelle application thérapeutique du Naftidrofuryl.

Exemple I

| Gélule renfermant 100 mg d'oxalate de Naftidrofuryl (LS 121) | |
|---|---|
| LS 121<br>Talc<br>Stéarate de magnésium | 100 mg<br>37 mg<br>3 mg |
| Gélule taille 4 : 1 gélule | 140 mg |

Exemple II

| Comprimé renfermant 300 mg d'oxalate de Naftidrofuryl. | |
|---|---|
| LS 121 | 300 mg |
| Copolymères de diméthyle aminoéthyle méthacrylate et d'esters neutres d'acide métacrylique | 100 mg |
| Phosphate tricalcique | 38 mg |
| Talc | 10 mg |
| Stéarate de magnésium | 2 mg |
| | 450 mg |

Exemple III

| Forme injectable à 200 mg de principe actif | |
|---|---|
| LS 121 | 200 mg |
| Sorbitol | 400 mg |
| eau p.p.i. | q.s.p. 10 ml |

Exemple IV

| Microgranules à 200 mg de principe actif | |
|---|---|
| LS 121 | 200 mg |
| Amidon de maïs | 15 mg |
| Gomme laque | 19 mg |
| Polyvidone excipient | 9,5 mg |
| Polyoxyéthylène glycol 4000 | 10 mg |
| Saccharose | 40 mg |
| Talc | 19 mg |
| Gélule taille 1 : 1 gélule. | 312,5 mg |

Exemple V

Etude pharmacologique.

La capacité de favoriser la croissance nerveuse que manifeste les sels d'addition de polyacides du Naftidrofuryl, tals que l'oxalate, le fumarate et le citrate, a été mise en évidence par différents tests, en particulier celui des ganglions spinaux de rat décrit ci-après.

Les essais sont réalisés sur ganglions spinaux lombaires et dorsaux prélevés au vingtième jour sur embryons de rat Wistar "in utéro".

Ces ganglions sont cultivés en tube de Leighyon sur lamelles recouvertes d'une pellicule de collagène reconstitué de queue de rat sous atmosphère normale en milieu Dulbecco modifié par N. Iscove et F. Melchers [J. Exp. Med, 147, 923 (1978)] additionné de 8 % de sérum de veau fétal et de glucose à raison de 6g par litre.

On procède à la numération et à la mesure de la longueur des prolongements nerveux et de la zone de croissance cellulaire émis en 48 heures de culture sous l'action du composé étudié utilisé à trois dilutions $10^{-6}$, $10^{-7}$ et $10^{-8}$ molaires, en comparison de cultures témoins.

Les observations sont effectuées au microscope après imprégnation par l'argent des cultures "in toto" selon la méthode de Bodian [Anat. Record. 69, 153-162 (1937)], montées sur lames codées.

Trois expériences comprenant chacune entre 15 et 25 ganglion dans les groupes expérimentaux et témoins ont été pratiquées.

On a rassemblé dans les tableaux I et II suivants les résultats pour les dilutions les plus faibles de $10^{-7}$

et $10^{-8}$ M; il apparait que ce composé est capable d'augmenter de façon statistiquement très significative par rapport aux témoins, le nombre de prolongements émis en 48 heures, le diamètre de la zone de croissance ainsi que la longueur des prolongements nerveux.

Il convient de souligner que la concentration optimale activ de $10^{-7}$ mole par litre d'oxalate de Naftidrofuryl est remarquablement faible et qu'elle reste inférieure à la concentration thérapeutique utile de ce composé en tant que vasodilatateur.

On obtient des résultats pratiquement similaires avec les sels d'addition du Naftidrofuryl , en particulier avec les sels de polyacides.

TABLEAU I : DONNEES BRUTES

| | Nombre de Ganglions | Moyennes | Déviations Standard | Nombre de Ganglions | Moyennes | Déviations Standard | Nombre de Ganglions | Moyennes | Déviations Standard |
|---|---|---|---|---|---|---|---|---|---|
| $T_1$ | 25 | 191,7 | 62,86 | 25 | 39,40 | 7,30 | 25 | 38,37 | 6,92 |
| $10^{-6}$ | 24 | 192,2 | 72,66 | 24 | 34,50 | 7,34 | 24 | 36,02 | 7,86 |
| $10^{-7}$ | 25 | 248,28 | 93,30 | 25 | 42,80 | 8,50 | 25 | 42,35 | 8,65 |
| $T_2$ | 20 | 109,90 | 68,35 | 20 | 24,37 | 5,45 | 20 | 20,95 | 4,64 |
| $10^{-7}$ | 18 | 142,90 | 58,56 | 18 | 31,56 | 7,27 | 17 | 28,80 | 7,34 |
| $10^{-8}$ | 16 | 133,93 | 54,38 | 16 | 29,40 | 6,30 | 16 | 26,35 | 6,28 |
| $T_3$ | 15 | 157,60 | 86,65 | 15 | 30,02 | 7,54 | 15 | 28,24 | 10,59 |
| $10^{-7}$ | 17 | 193,35 | 11,30 | 17 | 35,90 | 10,60 | 17 | 34,70 | 11,00 |
| $10^{-8}$ | 18 | 195,05 | 79,30 | 18 | 39,00 | 9,50 | 18 | 38,23 | 10,40 |
| | Nombre de prolongements | | | Longueur de la zone de croissance cellulaire | | | Longueur des prolongements | | |

**TABLEAU II : DONNEES CORRIGEES (COMPARAISON DES MOYENNES ET TEST DE STUDENT)**

| | Nombre de Ganglions | Nombre de prolongements | | | | Longueur de la zone de croissance | | | | Longueur des prolongements | | | |
|---|---|---|---|---|---|---|---|---|---|---|---|---|---|
| | | Moyennes | Déviations Standard | t | p | Moyennes | Déviations Standard | t | p | Moyennes | Déviation Standard | t | p |
| TEMOINS | 60 | 155,9 | 78,45 | | | 32,05 | 9,42 | | | 30,04 | 10,55 | | |
| $10^{-7}$ | 60 | 201,1 | 88,02 | 2,84 | 0,0054 | 37,76 | 9,43 | 3,32 | 0,0012 | 36,55 | 9,97 | 3,46 | 0,0004 |
| $10^{-8}$ | 34 | 219,9 | 110,12 | 3,27 | 0,0016 | 40,25 | 9,28 | 4,07 | <0,0001 | 39,30 | 10,12 | 4,15 | <0,0001 |

## Revendications

1. Utilisation du tétrahydro-α-(naphtyl-1 méthyl) furanne-2 propanoate de N,N-diéthylamino-2 éthyle, "Naftidrofuryl", pour l'obtention d'un médicament destiné à favoriser la régénération des fibres

nerveuses, notamment en vue du traitement des neuropathies.

2. Utilisation du Naftidrofuryl selon la revendication 1, pour l'obtention d'un médicament destiné à favoriser la régénération des fibres nerveuses en vue du traitement des neuropathies post-traumatiques.

3. Utilisation du Naftidrofuryl selon la revendication 1, pour l'obtention d'un médicament destiné à favoriser la régénération des fibres nerveuses en vue du traitement des neuropathies diabétiques.

4. Utilisation du Naftidrofuryl selon la revendication 1, pour l'obtention d'un médicament destiné à favoriser la régénération des fibres nerveuses en vue du traitement de certaines neuropathies toxiques, comme les polynévrites éthyliques.

5. Utilisation du Naftidrofuryl selon la revendication 1, pour l'obtention d'un médicament destiné à favoriser la régénération des fibres nerveuses pour rétablir ou améliorer la perception douloureuse au niveau cutané après intervention chirurgicale.

6. Utilisation du Naftidrofuryl selon la revendication 1, pour l'obtention d'un médicament destiné à favoriser la régénération des fibres nerveuses en vue du traitement des neuropathies d'origine bactérienne ou virale.

7. Utilisation du Naftidrofuryl selon la revendication 1, pour l'obtention d'un médicament destiné à favoriser la régénération des fibres nerveuses en vue du traitement des neuropathies dégénératives.

8. Utilisation du Naftidrofuryl selon l'une des revendications 1 à 7, caractérisée en ce que le Naftidrofuryl est sous forme de sel d'addition avec les acides pharmaceutiquement acceptables, choisis parmi les acides minéraux chlorhydrique, bromhydrique, iodhydrique, nitrique, sulfurique, phosphorique et les acides organiques formique, benzoique, fumarique, succinique, tartrique, citrique, oxalique, glyoxylique, aspartique, alcanesulfoniques et arylsulfoniques.

9. Utilisation du Naftidrofuryl selon l'une des revendications 1 à 7, caractérisée en ce que le Naftidrofuryl est sous forme de sel de polyacides organiques tels que l'oxalate, le fumarate et le citrate.

10. Utilisation du Naftidrofuryl selon l'une des revendications 1 à 9, caractérisé en ce que ledit médicament incorpore une quantité neurologiquement active de Naftidrofuryl comprise entre 0,100 et 1 g par dose unitaire.

11. Utilisation du Naftidrofuryl selon la revendication 10, caractérisée en ce que la quantité neurologiquement active de Naftidrofuryl est comprise entre 200 et 400 mg par dose unitaire.

12. Utilisation du Naftidrofuryl selon l'une des revendications 1 à 11, caractérisée en ce que le médicament favorisant la régénération des fibres nerveuses est sous forme de comprimés nus ou enrobés, dragées, microgranules, comprimés à plusieurs noyaux, gélules, capsules, poudres ou granulés, solutés, suspensions injectables, suppositoires ou capsules rectales.

## Claims

1. Use of tetrahydro-$\alpha$-(naphthyl-1 methyl) furan-2 propanoate of N,N-diethylamino-2 ethyl, "Naftidrofuryl", for obtaining a medicament for promoting the regeneration of nerve fibres, in particular with a view to the treatment of neuropathies.

2. Use of Naftidrofuryl according to claim 1, for obtaining a medicament for promoting the regeneration of nerve fibres with a view to the treatment of post-traumatic neuropathies.

3. Use of Naftidrofuryl according to claim 1, for obtaining a medicament for promoting the regeneration of nerve fibres with a view to the treatment of diabetic neuropathies.

4. Use of Naftidrofuryl according to claim 1, for obtaining a medicament for promoting the regeneration of

nerve fibres with a view to the treatment of certain toxic neuropathies such as alcoholic polyneuritis.

5. Use of Naftidrofuryl according to claim 1, for obtaining a medicament for promoting the regeneration of nerve fibres in order to reestablish or improve the perception of pain at the cutaneous level after surgical intervention.

6. Use of Naftidrofuryl according to claim 1, for obtaining a medicament for promoting the regeneration of nerve fibres with a view to the treatment of neuropathies of bacterial or viral origin.

7. Use of Naftidrofuryl according to claim 1, for obtaining a medicament for promoting the regeneration of nerve fibres with a view to the treatment of degenerative neuropathies.

8. Use of Naftidrofuryl according to one of claims 1 to 7, characterised in that the Naftidrofuryl is in the form of an addition salt with pharmaceutically acceptable acids chosen from among hydrochloric, hydrobromic, hydriodic, nitric, sulphuric, phosphoric mineral acids and formic, benzoic, fumaric, succinic, tartaric, citric, oxalic, glyoxylic, aspartic, alkane sulphonic and arylsulphonic organic acids.

9. Use of Naftidrofuryl according to one of claims 1 to 7, characterised in that the Naftidrofuryl is in the form of a salt of organic polyacids such as oxalate, fumarate and citrate.

10. Use of Naftidrofuryl according to one of claims 1 to 9, characterised in that the said medicament incorporates a neurologically active quantity of Naftidrofuryl of between 0.100 and 1 g per unit dose.

11. Use of Naftidrofuryl according to claim 10, characterised in that the neurologically active quantity of Naftidrofuryl is between 200 and 400 mg per unit dose.

12. Use of Naftidrofuryl according to one of claims 1 to 11, characterised in that the medicament promoting the regeneration of nerve fibres is in the form of non-coated or coated compressed pills, lozenges, microgranules, compressed pills having several cores, gelatine-coated pills, capsules, powders or granulates, solutions, injectable suspensions, suppositories or rectal capsules.

**Patentansprüche**

1. Verwendung von $\alpha$-(1-Naphthylmethyl)-2-tetrahydrofuranpropionsäure-2-N,N-diethylaminoethylester, "Naftidrofuryl", zur Gewinnung eines zur Förderung der Regenerierung von Nervenfasern, insbesondere bei der Behandlung von Neuropathien bestimmten Arzneimittels.

2. Verwendung von Naftidrofuryl nach Anspruch 1 zur Gewinnung eines zur Förderung der Regenerierung von Nervenfasern bei der Behandlung von posttraumatischen Neuropathien bestimmten Arzneimittels.

3. Verwendung von Naftidrofuryl nach Anspruch 1 zur Gewinnung eines zur Förderung der Regenerierung von Nervenfasern bei der Behandlung von Diabetesneuropathien bestimmten Arzneimittels.

4. Verwendung von Naftidrofuryl nach Anspruch 1 zur Gewinnung eines zur Förderung der Regenerierung von Nervenfasern bei der Behandlung bestimmter toxischer Neuropathien, wie von Ethylalkohol-Polyneuritiden, bestimmten Arzneimittels.

5. Verwendung von Naftidrofuryl nach Anspruch 1 zur Gewinnung eines zur Förderung der Regenerierung von Nervenfasern bestimmten Arzneimittels zur Wiederherstellung oder Verbesserung der Schmerzwahmehmung auf der Haut nach chirurgischem Eingriff.

6. Verwendung von Naftidrofuryl nach Anspruch 1 zur Gewinnung eines zur Förderung der Regenerierung von Nervenfasern bei der Behandlung von Neuropathien mit Bakterien- oder Virenursprung bestimmten Arzneimittels.

7. Verwendung von Naftidrofuryl nach Anspruch 1 zur Gewinnung eines zur Förderung der Regenerierung von Nervenfasern bei der Behandlung von degenerativen Neuropathien bestimmten Arzneimittels.

8. Verwendung von Naftidrofuryl nach einem der Ansprüche 1 bis 7, **dadurch gekennzeichnet,** daß das Naftidrofuryl in der Form eines Additionssalzes mit den pharmazeutisch verträglichen Säuren vorliegt, die unter den Mineralsäuren Salzsäure, Bromwasserstoffsäure, Jodwasserstoffsäure, Salpetersäure, Schwefelsäure und Phosphorsäure und unter den organischen Säuren Ameisensäure, Benzoesäure, Fumarsäure, Bernsteinsäure, Weinsäure, Zitronensäure, Oxalsäure, Glyoxalsäure, Alkansulfonsäuren und Arylsulfonsäuren ausgewählt sind.

9. Verwendung von Naftidrofuryl nach einem der Ansprüche 1 bis 7, **dadurch gekennzeichnet,** daß das Naftidrofuryl in der Form eines Salzes organischer Polysäuren, wie als das Oxalat, Fumarat und Citrat, vorliegt.

10. Verwendung von Naftidrofuryl nach einem der Ansprüche 1 bis 9, **dadurch gekennzeichnet,** daß das Arzneimittel eine neurologisch wirksame Menge von Naftidrofuryl zwischen 0,100 und 1 g je Einheitsdosis enthält.

11. Verwendung von Naftidrofuryl nach Anspruch 10, **dadurch gekennzeichnet,** daß die neurologisch wirksame Menge von Naftidrofuryl zwischen 200 und 400 mg je Einheitsdosis liegt.

12. Verwendung von Naftidrofuryl nach einem der Ansprüche 1 bis 11, **dadurch gekennzeichnet,** daß das die Regenerierung von Nervenfasern fördernde Arzneimittel in der Form von nackten oder umhüllten Tabletten, Dragees, Mikrogranulat, mehrkernigen Tabletten, Pastillen, Kapseln, Pulvern oder Körnern, in Lösungen, injizierbaren Suspensionen, Suppositorien oder Rektalkapseln vorliegt.